# EUROPÄISCHE PATENTSCHRIFT

(11) **EP 1 002 784 B1**
(45) Veröffentlichungstag und Bekanntmachung des Hinweises auf die Patenterteilung: **06.02.2002**
(21) Anmeldenummer: 99120113.8
(22) Anmeldetag: 08.10.1999
(51) Int. Cl.: C07C 45/50

(54) **Verfahren zur Hydroformylierung eines Alkens**
Process for hydroformylating alkenes
Procédé pour l'hydroformylation d'alcènes

(30) Priorität: 19.11.1998 DE 19853371
(43) Veröffentlichungstag der Anmeldung: 24.05.2000
(73) Patentinhaber: Forschungszentrum Karlsruhe GmbH, 76133 Karlsruhe (DE)
(72) Erfinder: Dinjus, Eckhard, Prof. Dr., 76774 Leimersheim (DE); Riffel, Werner, 76689 Karlsdorf-Neuthard (DE); Borwieck, Henning, Dr., 25709 Kaiser-Wilhelm-Koog (DE)

(56) Entgegenhaltungen:
- US-A- 5 198 589
- CHEMICAL ABSTRACTS, vol. 131, Columbus, Ohio, US; abstract no. 228825, BORWIECK H: "Cyclotrimerizations and oxo synthesis in supercritical water" XP002129764 & WISS. BER. - FORSCHUNGSZENT. KARLSRUHE (WBFKF5,09478620);1999; (FZKA 6302,); 104 PP., Forschungszentrum Karlsruhe G.m.b.H.;Inst. Technische Chem., Projekt Schadstoff- Abfallarme Verfahren; Karlsruhe; D-76021; Germany (DE)

## Beschreibung

Die Erfindung betrifft ein Verfahren zur Hydroformylierung eines Alkens gemäß dem Oberbegriff des ersten Patentanspruchs.

Unter "Hydroformylierung" wird die Umsetzung eines Alkens mit Kohlenmonoxid und Wasserstoff gemäß der Formel 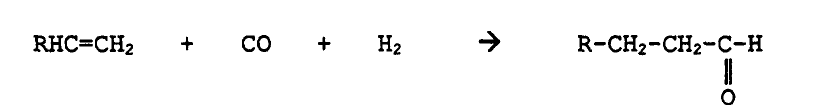 verstanden, wobei n- oder iso-Aldehyde entstehen, die ein Kohlenstoffatom mehr aufweisen als das eingesetzte Alken. In Folgereaktionen kann der Aldehyd zu dem entsprechenden Alkohol reduziert werden. Häufig wird anstelle des Begriffs Hydroformylierung synonym die Bezeichnung "Oxo-Synthese" verwendet. R ist meist ein Alkylrest wie z. B. Methyl; Arylreste oder Alkylreste mit Arylsubstituenten sind jedoch ebenfalls einsetzbar. Die Reaktion wird konventionell üblicherweise unter Druck (30 bis 300 bar) und erhöhter Temperatur (100°C bis 180°C) durchgeführt (CD Römpp Chemie Lexikon - Version 1.0, Stuttgart/New York: Georg Thieme Verlag 1995, Stichworte: "Hydroformylierung" und "Oxo-Synthese").

Das Alken kann unmittelbar oder gelöst in einem organischen Lösungsmittel eingesetzt werden. Als Katalysatoren sind prinzipiell Verbindungen von Metallen der 8., 9., 10. oder 11. Nebengruppe des Periodischen Systems gemäß der IUPAC-Nomenklatur 1989 einsetzbar, insbesondere Hydridocarbonylverbindungen von Eisen, Kobalt, Nickel, Kupfer und der in den jeweiligen Nebengruppen nachfolgenden Metalle. Man kann die Katalysatoren in Einphasen- und Zweiphasensysteme einteilen. Katalysatoren für Einphasensysteme sind beispielsweise HRh(CO)₄, HRh(CO)₃(PPh₃) mit Ph=Phenyl, HCo(CO)₄ und HCo(CO)(PPh₃)₃, die in situ durch Reaktion von Metallcarbonyl - der Katalysatorvorstufe - mit Wasserstoff gebildet werden. Diese Katalysatoren sind in organischer Phase leicht löslich; ein Nachteil ist, daß die Abtrennung der entstehenden organischen Produkte von den Katalysatorrückständen durch Destillation erfolgen muß. Bei Zweiphasensystemen werden unlösliche Katalysatoren z. B. durch starkes Rühren in einen quasi homogenen Zustand überführt, der für die Produkttrennung wieder aufgehoben wird. Nachteilig sind die verminderten Ausbeuten (Internet-Artikel http://www.gwdg.de/∼hroesky/tb/be7.htm).

Ein Verfahren der eingangs genannten Art ist in der Veröffentlichung von Yang Guo und Aydin Akgerman: "Hydroformylation of Propylene in Supercritical Carbon Dioxide", Ind. Eng. Chem.Res. **1997**, *36*, 4581-4585 beschrieben. Nach diesem Verfahren wird Propylen in einem Reaktor bei einer Temperatur zwischen 66°C und 108°C und einem Überdruck von 93 bis 186 bar in überkritischem Kohlendioxid hydroformyliert, wobei sich das als Katalysatorvorstufe eingesetzte Co₂(CO)₈ und der daraus entstehende, katalytisch aktive Hydridokomplex im überkritischen Kohlendioxid homogen lösen läßt. In dem Reaktor wurde Propylen vorgelegt und Kohlenmonoxid und Wasserstoff im Volumenverhältnis 1:1 bei einem Druck von ungefähr 168 bar und einer Temperatur von 88°C aufgepreßt. Anschließend wurden durch Zugabe von Kohlendioxid die Betriebsparameter eingestellt.

In zwei Veröffentlichungen von Rathke et al (J. W. Rathke, R. J. Klingler: "Propylene Hydroformylation in Supercritical Carbon Dioxide", Organometallics **1991**, *10*, 1350-1355 und J. W. Rathke, R. J. Klingler und T. R. Krause: "Thermodynamics for the Hydrogenation of Dicoblat Octacarbonyl in Supercritical Carbon Dioxide", Organometallics **1992**, *11*, 585-588) werden die Eigenschaften und Vorteile des überkritischen Lösungsmittels Kohlendioxid ausführlich dargestellt (siehe auch US-A-5 198 589).

Überkritisches Kohlendioxid ist ein verhältnismäßig teures Lösungsmittel. Hinsichtlich der wirtschaftlichen Einsatzmöglichkeit bei der Hydroformylierung von Alkenen ist zwar von Vorteil, daß Kohlendioxid unter dem entsprechenden Druck (p_{kritisch} = 73,8 bar) bereits bei relativ geringen Temperaturen (T_{kritisch} = 31°C) in den überkritischen Zustand Übergeht; im Interesse höherer Ausbeuten und eines schnelleren Reaktionsablaufes werden jedoch ohnehin wesentlich höhere Reaktionstemperaturen eingestellt.

Der Erfindung liegt die Aufgabe zugrunde, ein weiteres überkritisches Lösungsmittel für ein Verfahren der eingangs genannten Art vorzuschlagen, das kostengünstiger ist, wobei die mit überkritischem Kohlendioxid als Lösungsmittel erzielbaren Vorteile erhalten bleiben sollen.

Die Lösung der Aufgabe ist im ersten Patentanspruch gekennzeichnet. Die weiteren Patentansprüche geben bevorzugte Ausgestaltungen des erfindungsgemäßen Verfahrens an.

Erfindungsgemäß wird als Lösungsmittel für die Hydroformylierung von Alkenen überkritisches Wasser vorgeschlagen. Erfindungsgemäß wird die Hydroformylierung somit bei Temperaturen über T_{kritisch} = 374°C und Drücken von mindestens p_{kritisch} = 220,5 bar durchgeführt. Temperaturen über 500°C und Drücke über 400 bar sind in der Regel nicht erforderlich und daher unwirtschaftlich. Wie sich gezeigt hat, können die üblicherweise eingesetzten Katalysatorvorstufen und die in situ entstehenden Katalysatoren in überkritischem Wasser in gleicher Weise wie in überkritischem Kohlendioxid homogen in einer einzigen Phase gelöst werden, wobei die Aktivität des Katalysators vollständig erhalten bleibt. Dies ist deswegen überraschend, weil Hydridocarbonylkomplexe wie z. B. HCo(CO)₄ in wäßriger Lösung schwer löslich sind und das Proton nahezu vollständig dissoziiert (Milton Orchin: "HCo(CO)₄, the Quintessential Catalyst", Accounts of Chemical Research, Vol. 14, No. 9 (September 1981) 259-266) und weil Übergangsmetallcarbonyle generell bei höherer Temperatur in Metall und Kohlenmonoxid zerfallen (vgl. z. B. Hofmann und Rüdorff, Anorganische Chemie, F. Vieweg & Sohn Braunschweig,19. Aufl. (1966), Seite 757).

Ein wesentlicher Vorzug von überkritischem Wasser als Lösungsmittel ist, daß im Gegensatz zu den bekannten Hydroformylierungen auf die Zugabe von Wasserstoff verzichtet werden kann. Der für die Hydroformylierung benötigte Wasserstoff entsteht in ausreichender Menge unmittelbar aus dem Lösungsmittel. Der Verzicht auf die Wasserstoffeinspeisung stellt einen erheblichen Sicherheitsgewinn bei der Durchführung der Reaktion dar.

Ein weiterer wesentlicher Vorteil von überkritischem Wasser als Lösungsmittel ist, daß die Dielektrizitätskonstante in Abhängigkeit von Temperatur und Druck variiert werden kann. Bei konstantem Druck nimmt die Dieelektrizitätskonstante von überkritischem Wasser mit steigender Temperatur ab, während sie bei vorgegebener Temperatur mit steigendem Druck steigt. Die Löslichkeiten des eingesetzten Alkens und des Katalysators können somit durch geeignete Wahl von Temperatur und Druck beeinflußt werden. Dies ist bei überkritischem Kohlendioxid nicht der Fall.

In überkritischem Wasser können sowohl aliphatische als auch alizyklische Alkene wie z. B. Cyclohexen hydroformyliert werden. Die Alkene können aliphatische oder aromatische Substituenten tragen, wobei aliphatische Substituenten ihrerseits Arylreste aufweisen können. Terminale Alkene können sowohl am terminalen als auch am benachbarten Kohlenstoffatom hydroformyliert werden. Das n/iso-Verhältnis bei den Reaktionsprodukten läßt sich durch geeignete Wahl der Reaktionsparameter einstellen.

Die Hydroformylierung kann erfindungsgemäß in drei Verfahrensschritten durchgeführt werden. Im ersten Verfahrensabschnitt wird eine wäßrige Phase, die den Katalysator oder die Katalysatorvorstufe in gelöster oder dispergierter Form enthält, mit der organischen Phase des Alkens überschichtet, wonach Kohlenmonoxid und gegebenenfalls Wasserstoff aufgepreßt wird. Danach werden die überkritischen Bedingungen für Wasser eingestellt, wobei die drei Phasen - Gas, organische und wäßrige Phase - in eine einzige Reaktionsphase überführt werden. Nach dem Abkühlen liegen wieder drei Phasen vor: eine CO₂-angereicherte Gasphase, eine organische Produktphase und eine wäßrige Phase, die den Katalysatorrückstand enthält. Das Produkt kann somit in einfacher Weise und ohne Destillation durch Phasentrennung vom Katalysatorrückstand getrennt werden, wonach der Katalysatorrückstand wieder aufgearbeitet werden kann. Dies ist ein weiterer wesentlicher Vorzug von überkritischem Wasser gegenüber überkritischem Kohlendioxid als Lösungsmittel. Bei der Verwendung von überkritischem Kohlendioxid als Lösungsmittel bleibt nach der Beendigung der Reaktion und dem Entfernen des Kohlendioxids nur eine flüssige organische Produktphase zurück, die den Katalysatorrückstand enthält. Zur Reinigung der Produkte ist die Entfernung des Katalysatorrückstands erforderlich.

Die Erfindung wird im folgenden anhand von Ausführungsbeispielen näher erläutert.

Die Versuche wurden in einem 5 ml-Reaktor aus Inconel 625 mit Gasanschlüssen für Argon, Wasserstoff und Kohlendioxid durchgeführt. Die Edukte 1-Hexen und Cyclohexen wurden zuvor nach den Standard-Methoden destilliert und getrocknet. Kohlenmonoxid (Messer-Griesheim, 3.7) und Wasserstoff (Linde, 5.0) wurden ohne Vorbehandlung eingesetzt. Um reproduzierbare Ergebnisse zu erhalten, wurde das als Lösungsmittel eingesetzte Wasser vor der Verwendung unter Argon-Atmosphäre 48 h im Rückfluß gekocht. Die Katalysatorvorstufe Co₂(CO)₈ (Octacarbonyldicobalt, Fluka, 90-95 %, mit 5-10% n-Hexan als Stabilisator) wurde unter Argon in einem Schlenk-Rohr aufbewahrt. Die Katalysatorvorstufe [Co(CO)₃PPh₃]₂ wurde durch Umsetzung von Co₂(CO)₈ mit zwei Moläquivalenten PPh₃ hergestellt. Die Charakterisierung der Produkte erfolgte durch GC-MS-Spektroskopie. Die Untersuchungen an [Co(CO)₃PPh₃]₂ erfolgte ³¹P-NMR-und IR-spektroskopisch.

Zur Hydroformylierung wurde eine Mischung von 0,3 ml (0,2 g, 2,4 mmol) 1-Hexen, 5 µl n-Oktan als interner Standard, 0,04 g entsprechend 0,115 mmol Co₂(CO)₈ und Wasser bzw. eine Mischung von 0,25 ml (0,2 g, 2,4 mmol) Cyclohexen, 5 µl n-Oktan, 0,1 g entsprechend 0,115 mmol [Co(CO)₃PPh₃]₂ und Wasser mit Standard-Schlenk-Verfahren in den Reaktor überführt. Wasserstoff und Kohlenmonoxid wurden in das System eingespeist und mit einer Schraubenpresse auf den entsprechenden Druck eingestellt, bis der gewünschte Vordruck erreicht war. Danach wurde der Reaktor auf 380°C bei 250 bar aufgeheizt. Nach 30 Minuten wurde der Reaktor auf Raumtemperatur abgekühlt und auf Normaldruck gebracht. Die Gasphase wurde mit GC-Techniken untersucht. Die flüssige organische Phase wurde von der flüssigen wäßrigen Phase, in der der Katalysatorrückstand enthalten war, abgetrennt. Die wäßrige Phase wurde nach Abtrennung des Katalysatorrückstandes wieder eingesetzt.

### Beispiel 1

### Hydroformylierung von Cyclohexen mit Co₂(CO)₈ bei 380°C und 250 bar

| **Exp. Nr.** | **Co**_{**2**}**(CO)**_{**8**} **[mmol]** | **p(H**_{**2**}**) [bar]** | **P(CO) bar** | **Umsatz C**_{**6**}**H**_{**10**} | **Ausbeute C**_{**6**}**H**_{**12**} | **Ausbeute C**_{**7**}**-Aldehyd** | **Ausbeute C**_{**7**}**-Alkohol** |
|---|---|---|---|---|---|---|---|
| 1 | 0,115 | 0 | 0 | 11 | 0 | 93 | 7 |
| 2 | 0,115 | 25 | 35 | 43 | 4 | 78 | 16 |
| 3 | 0,115 | 0 | 45 | 31 | 8 | 48 | 42 |
| 4 | 0,115 | 0 | 35 | 33 | 7 | 58 | 33 |
| 5 | 0,115 | 0 | 10 | 36 | 6 | 75 | 17 |
| 6 | 0,115 | 0 | 5 | 40 | 4 | 84 | 10 |
| 7 | 0 | 25 | 35 | 0 | 0 | 0 | 0 |
| 8 | 0,0115 | 0 | 5 | 1,5 | 0 | 66 | 33 |

Die Ausbeuten sind in [%] angegeben und basieren auf dem umgesetzten Cyclohexen. 1-5% des umgesetzten Cyclohexens ergaben undefinierbare Produkte.

Wie aus den Experimenten Nr. 1, 3-6 und 8 hervorgeht, findet die Hydroformylierung auch ohne Zusatz von Wasserstoff statt. Mit abnehmendem CO-Druck steigen die Ausbeuten an Aldehyd an und die Alkohol-Ausbeuten sinken. Der Gesamtanteil an umgesetztem Cyclohexen steigt mit abnehmender CO-Konzentration. Bei abnehmendem CO-Druck werden geringere Anteile von Cyclohexan und Alkohol gebildet. Die höchsten Umsatzraten wurden mit gleichzeitig hohen CO- und H₂-Konzentrationen unter Zusatz von Wasserstoff erzielt. Ohne Katalysatorvorstufe fand kein Umsatz statt. Niedrigere Mengen an Katalysatorvorstufe ergaben geringere Umsatzraten von Cyclohexen.

### Beispiel 2

### Hydroformylierung von 1-Hexen mit Co₂(CO)₈ bei 380°C und 250 bar

| **Exp. Nr.** | **Co**_{**2**}**(CO)**_{**8**} **[mmol]** | **p(H**_{**2**}**) [bar]** | **p(CO) [bar]** | **(5)** | **(6)** | **(7)** | **(8)** | **(9)** | **(10)** | **(11)** | **(12)** |
|---|---|---|---|---|---|---|---|---|---|---|---|
| 1 | 0,115 | 0 | 5 | 92 | 1 | 66 | 13 | 1,5 | 6 | 61 | 39 |
| 2 | 0,115 | 25 | 35 | 97 | 1 | 37 | 42 | 10 | 3 | 60 | 40 |
| 3 | 0 | 25 | 35 | 0 | 0 | 0 | 0 | 0 | 0 | | |

| | | | | | | | | | | | |
|---|---|---|---|---|---|---|---|---|---|---|---|
| Spalte (5): Umsetzung von 1-Hexen in [%] | | | | | | | | | | | |
| Spalte (6): Ausbeute an n-Hexan in [%] | | | | | | | | | | | |
| Spalte (7): Ausbeute an iso-Hexen in [%] | | | | | | | | | | | |
| Spalte (8): Ausbeute an C₇-Aldehyd in [%] | | | | | | | | | | | |
| Spalte (9): Ausbeute an C₇-Alkohol in [%] | | | | | | | | | | | |
| Spalte (10): Ausbeute an C₁₃-Keton in [%] | | | | | | | | | | | |
| Spalte (11): Ausbeute an linearen Produkten in [%] | | | | | | | | | | | |
| Spalte (12): Ausbeute an verzweigten Produkten in [%]. | | | | | | | | | | | |

Die Werte in Spalte (11) und (12) beziehen sich auf die Gesamtausbeute an C₇-Aldehyden und -Alkoholen. 3 bis 8 % des 1-Hexens reagierten zu undefinierten Produkten.

### Beispiel 3

### Hydroformylierung von 1-Hexen mit verschiedenen Katalysatorvorstufen bei 380°C und 250 bar

Es wurden zwei Versuche durchgeführt. Im ersten Versuch wurden 0,115 mmol der Katalysatorvorstufe [Co(CO)₃PPh₃]₂ und im zweiten Versuch 0,115 mmol der Katalysatorvorstufe Co₂(CO)₈ eingesetzt. In beiden Versuchen wurde ein Gemisch aus Wasserstoff und Kohlenmonoxid mit einem Gesamtdruck von 35 bar eingesetzt; 25 bar der Gesamtmenge wurden mit Wasserstoff eingestellt, Kohlenmonoxid wurde bis auf 35 bar mit der Schraubenpresse zugefügt. In den beiden Versuchen wurden folgende Umsätze bzw. Ausbeuten in [%] erzielt:

| **Umsatz 1-Hexen [%]** | **n-Hexan [%]** | **iso- Hexen [%]** | **C**_{**7**}**- Aldehyd** | **C**_{**7**}**- Alkohol** | **C**_{**13**}**- Keton** | **lineare Produkte** | **iso- Produkte** |
|---|---|---|---|---|---|---|---|
| 79 | 1 | 64 | 5 | 2 | 0 | 68 | 32 |
| 97 | 1 | 37 | 42 | 10 | 3 | 60 | 40 |

3 bis 8 % des 1-Hexens reagierten zu undefinierten Produkten.

## Patentansprüche

1. Verfahren zur Hydroformylierung eines Alkens, bei dem
a) ein in einem überkritischen Lösungsmittel gelöstes Alken eingesetzt wird,
b) das Alken mit Kohlenmonoxid und Wasserstoff in Gegenwart eines Katalysators umgesetzt wird,
**dadurch gekennzeichnet, daß**
c) als überkritisches Lösungsmittel Wasser eingesetzt wird.

2. Verfahren nach Anspruch 1,
**dadurch gekennzeichnet, daß**
auf den Zusatz von Wasserstoff verzichtet wird.

3. Verfahren nach Anspruch 1 oder 2,
**dadurch gekennzeichnet, daß**
als Alken ein aliphatisches oder ein alizyklisches Alken eingesetzt wird.

## Claims

1. Method of hydroformylating an alkene, wherein
a) an alkene, which is dissolved in a supercritical solvent, is used, and
b) the alkene is reacted with carbon monoxide and hydrogen in the presence of a catalyst,
**characterised in that**
c) water is used as the supercritical solvent.

2. Method according to claim 1, **characterised in that** there is no addition of hydrogen.

3. Method according to claim 1 or 2, **characterised in that** an aliphatic alkene or an alicyclic alkene is used as the alkene.

## Revendications

1. Procédé d'hydroformylation d'un alcène, dans lequel :
a) on met en oeuvre un alcène dissout dans un solvant supercritique,
b) on fait réagir l'alcène avec du monoxyde de carbone et de l'hydrogène en présence d'un catalyseur
**caractérisé en ce que**
c) comme solvant supercritique on utilise de l'eau.

2. Procédé selon la revendication 1,
**caractérisé en ce qu'**
on renonce à l'addition d'hydrogène.

3. Procédé selon la revendication 1 ou la revendication 2,
**caractérisé en ce qu'**
on utilise comme alcène, un alcène aliphatique ou un alcène alicyclique.
